# EUROPEAN PATENT APPLICATION

(11) **EP 3 206 146 A1**
(43) Date of publication of application: **16.08.2017**
(21) Application number: 16758075.2
(22) Date of filing: 01.07.2016
(51) Int. Cl.: G06F 19/00, G06F 17/30

(54) **DATA INFORMATION PROCESSING METHOD AND DEVICE**

(30) Priority: 14.12.2015 CN 201510927475
(71) Applicant: LE Holdings (Beijing) Co., Ltd., Beijing 100025 (CN); Le Shi Internet Information & Technology Corp., Beijing, Beijing 100025 (CN)
(72) Inventor: LIU, Hongbin, Beijing 100025 (CN); GUO, Tielong, Beijing 100025 (CN)
(74) Representative: DREISS Patentanwälte PartG mbB
(86) International application number: PCT/CN2016/088128
(87) International publication number: WO 2017/101301

(57) **Abstract**

This disclosure relates to a method and device for data information processing. The method may include: acquiring task logic of data processing when the data is under processing; determining input data and output data corresponding to the task logic according to executing logic of the task logic; determining data DNA relation information between the input data and the output data; and establishing, with respect to a plurality of stored data, a data DNA relation network among the plurality of stored data, according to the data DNA relation information of each of the stored data. With such a method, if certain data in the network is problematic, other suspected problematic data may be found through the data DNA relation network, so as to allow the technician to remove or modify the data having quality problem, and improve the quality of the stored data.

## Description

### CROSS REFERENCE TO RELATED APPLICATION

This disclosure claims priority to a Chinese Patent Application No. 201510927475.X, entitled "Method And Device For Data Information Processing", filed with the State Intellectual Property Office of the PRC on December 14, 2015, the disclosure of which is incorporated herein by reference in its entirety.

### TECHNICAL FIELD

The present disclosure relates to the field of data information processing, and in particular, to a method and device for data information processing.

### BACKGROUND

Data is one of the most valuable properties for an organization. For an enterprise, the data quality may be directly associated with business performance. High-quality of data may always strengthen the competiveness of a company and bring advantages to it even in times of economic instability. With broad and deep data quality, managers may have their trust in all data that satisfies all their needs all the time.

Data quality management includes a series of management activities including identification, measurement, monitoring and early warning on various data quality problems which may occur in each phases in data life cycle including planning, acquisition, storage, sharing, maintenance, application and extinction, and data quality can be improved further by improving the management level of the organization.

Currently, in data quality management, massive data is still inputted manually, which may form a relatively sparse distribution of different data. However, in the actual data generation process, there may be some relevance between different data, such that when dealing with massive data, if certain data is found to have quality problem, other data associated with the data having quality problem may not be found effectively in time, which may form potential threats to the data and deteriorate the data quality.

### SUMMARY

In order to solve the above issues in related art, this disclosure provides a method and device for data information processing.

According to a first aspect of the embodiment of this disclosure, a method for data information processing is provided, the method includes:
Acquiring task logic of data processing when the data is under processing;
Determining input data and output data corresponding to the task logic according to executing logic of the task logic;
Determining data DNA relation information between the input data and the output data; and
With respect to a plurality of stored data, establishing a data DNA relation network among the plurality of stored data according to the data DNA relation information of each of the stored data.

Alternatively, the data DNA relation information may include at least a data flow direction;

The task logic may include any one of data format conversion and data operation.

Alternatively, establishing the data DNA relation network among the plurality of stored data may include:
Generating a node diagram including the plurality of stored data, each of the stored data being presented in form of node in the node diagram;
Determining whether the a data DNA relation exists between any two of the nodes in the node diagram; and
Indicating a data flow direction of the two nodes between the two nodes according to the data DNA relation information if the data DNA relation exists between the two nodes.

Alternatively, the method may further include:
Acquiring a target data DNA relation network to which the problematic data with quality problem belongs when the problematic data is detected;
Searching for all data that has data DNA relation with the problematic data in the target data DNA relation network; and
Determining the data found as suspected problematic data.

Alternatively, the method may further include:
Determining whether the data processing for first data in the data DNA relation network is detected;
Acquiring data DNA relation information of second data associated with the first data according to current task logic if the data processing for first data in the data DNA relation network is detected; and
Adding a node corresponding to the second data in the data DNA relation network according to the data DNA relation information of the second data.

According to a second aspect of the embodiment of this disclosure, a device for data information processing is provided, which includes:
A task logic acquiring module, configured to acquire task logic of data processing when the data is under processing;
An associated data determining module, configured to determine input data and output data corresponding to the task logic according to executing logic of the task logic;
A data DNA relation information determining module, configured to determine data DNA relation information between the input data and the output data; and
A network establishing module, configured to, with respect to a plurality of stored data, establish a data DNA relation network among the plurality of stored data according to the data DNA relation information of each of the stored data.

Alternatively, the data DNA relation information may include at least a data flow direction;

The task logic may include any one of data format conversion and data operation.

Alternatively, the network establishing module may include:
A node diagram generating submodule, configured to generate a node diagram including the plurality of stored data, each of the stored data being presented in form of node in the node diagram;
A data DNA relation determining submodule, configured to determine whether the a data DNA relation exists between any two of the nodes in the node diagram; and
A data flow direction indicating submodule, configured to indicate a data flow direction of the two nodes between the two nodes according to the data DNA relation information if the data DNA relation exists between the two nodes.

Alternatively, the device may further include:
A first data DNA relation network acquiring module, configured to acquire a target data DNA relation network to which problematic data with quality problem belongs when the problematic data is detected;
A data searching module, configured to search for all data that has data DNA relation with the problematic data in the target data DNA relation network; and
A suspected problematic data determining module, configured to determine the data found as suspected problematic data.

Alternatively, the device may further include:
A data processing determining module, configured to determine whether the data processing for first data in the data DNA relation network is detected;
A second data DNA relation information acquiring module, configured to acquire data DNA relation information of second data associated with the first data according to the current task logic, if the data processing for the first data in the data DNA relation network is detected; and
A node adding module, configured to add a node corresponding to the second data in the data DNA relation network, according to the data DNA relation information of the second data.

According to a third aspect of the embodiment of the disclosure, a computer storage medium is provided, wherein, the computer storage medium may be stored with program, some or all steps in the embodiments of the method for data information processing provided in the first aspect of the disclosure may be implemented when the program is executed.

The technical solutions provided in the embodiments of this disclosure may have the following advantageous effects:
The method according to the embodiments of the disclosure may include: acquiring task logic of data processing when the data is under processing; determining input data and output data corresponding to the task logic according to executing logic of the task logic; determining data DNA relation information between the input data and the output data; and establishing, with respect to a plurality of stored data, a data DNA relation network among the plurality of stored data according to the data DNA relation information of each of the stored data.

In this method, for any one of processed data, the input data and the output data associated with task logic may be determined by acquiring executing logic corresponding to the task logic. Because the task logic exists between the input data and the output data, it may be determined that the input data and the output data have a data DNA relation, and then, for each of the stored data, the data DNA relation among data may be determined through the method, and the data DNA relation network of all stored data may be acquired at last. With such a method, if certain data in the network is problematic, other suspected problematic data may be found through the data DNA relation network, so as to allow the technician to remove or modify data having quality problem, and improve the quality of the stored data.

It should be understood that the above general description and detailed description below are only exemplary and illustrative, rather than to limit the disclosure.

### BRIEF DESCRIPTION OF THE DRAWINGS

The drawings attached here are incorporated into the specification and form a part of the specification, illustrate the embodiments of the disclosure, and used to explain the principle of the disclosure along with the description.

In order to illustrate the embodiments of the disclosure or the technical solutions in prior art more clearly, a brief introduction will be given below for the drawings in the embodiments of the disclosure or the description of prior art. Apparently, other drawings may be obtained based on the following drawings for one skilled in the art without inventive effort.
Figure 1 is a flowchart illustrating a method for data information processing according to an embodiment of the disclosure;
Figure 2 is a schematic diagram illustrating Step S103 in Figure 1;
Figure 3 is a schematic diagram illustrating nodes according to an embodiment of the disclosure;
Figure 4 is a schematic diagram illustrating a data DNA relation network according to an embodiment of the disclosure;
Figure 5 is a flowchart illustrating another method for data information processing according to an embodiment of the disclosure;
Figure 6 is a flowchart illustrating still another method for data information processing according to an embodiment of the disclosure;
Figure 7 is a schematic diagram illustrating another data DNA relation network according to an embodiment of the disclosure;
Figure 8 is a structural schematic illustrating a device for data information processing according to an embodiment of the disclosure;
Figure 9 is a structural schematic illustrating the network establishing module in Figure 8;
Figure 10 is a structural schematic illustrating another device for data information processing according to an embodiment of the disclosure; and
Figure 11 is a structural schematic illustrating still another device for data information processing according to an embodiment of the disclosure.

### DETAILED DESCRIPTION

Embodiments will be illustrated in detail herein, examples of which are illustrated in the drawings. In the following description of the drawings, when regarding the drawings, the same numerals in different drawings represent the same or similar element unless otherwise indicated. The embodiments described in the following embodiments do not represent all embodiments corresponding to the disclosure, but rather, they are examples of the device and method corresponding to some aspects of the disclosure which are described in attached claims.

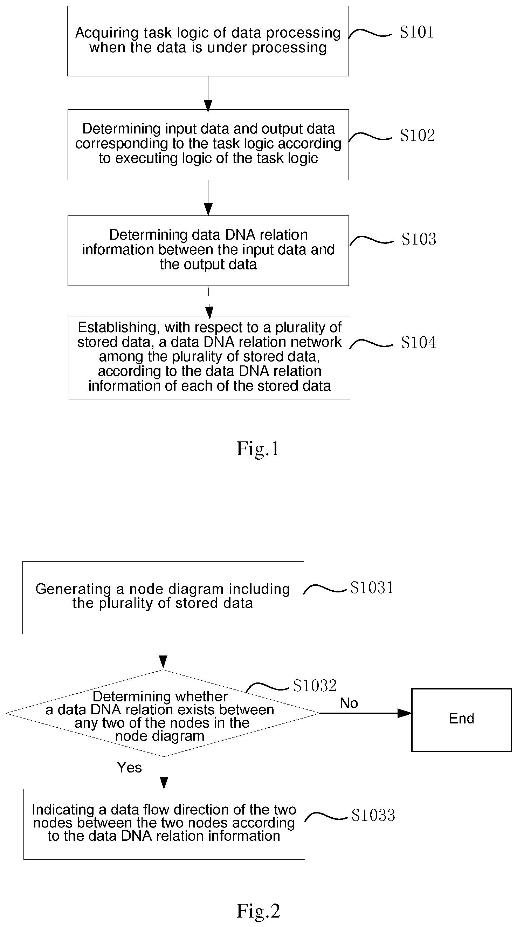

Figure 1 is a flowchart illustrating a method for data information processing according to an embodiment of the disclosure. The method for data information processing method can be applied in a server. As shown in Figure 1, the method may include the following steps.

In step S101: task logic for data processing is acquired when the data is under processing.

In different storage media, data may be in different forms. For convenience of description, data is described in a form of data unit in the embodiment of the disclosure. Generally, the output target for each of data processing tasks may be regarded as one data unit. Further, the input source for each of data processing tasks may be regarded as one data unit. For example, in a relational database, each of the cells in a data table may be regarded as one data unit. Additionally, in a front-end display, the report form may be also regarded as one data unit.

Based on the above description, in this step, the task logic for data processing on a data unit may be acquired by taking the data unit as an object.

In the embodiment of the disclosure, the task logic refers to data processing way, such as data format conversion, data operation, etc.

In step S102: input data and output data corresponding to the task logic are determined according to executing logic of the task logic.

Each task logic has performing logic. In the embodiment of the disclosure, the executing logic refers to a flow direction between data. Taking data format conversion as an example, data before format conversion is input data, and data after format conversion is output data. Taking data operation as an example, each of parameters before data operation is input data, and data after data operation is output data.

In step S103: data DNA relation information between the input data and the output data is determined;

Because both the input data and the output data are associated with the same task logic, the output data is acquired after data processing on the input data. Thus, it can be determined that correlation exists between the input data and the output data.

In the embodiment of the disclosure, data DNA relation information includes at least a data flow direction.

In step S104: with respect to a plurality of stored data, a data DNA relation network among the plurality of stored data is established according to the data DNA relation information of each of the stored data.

For each of the stored data recorded in the server, data DNA relation information corresponding to the stored data can be acquired. In this way, in the process of accumulation of stored data, the data DNA relation information of each of stored data can be acquired simultaneously. The flow direction relation between the stored data can be acquired through the data DNA relation information. Therefore, in this step, on basis of the flow direction relation between the stored data, relations among all stored data can be figured out to acquire the data DNA relation network among the plurality of stored data, that is, a "family tree" among data, a parent node (that is the input data) and a child node (that is the output data) for each of the nodes may be found according to the "family tree".

The method according to the embodiment of the disclosure may include: acquiring task logic for data processing when the data is under processing; determining input data and output data corresponding to the task logic according to executing logic of the task logic; determining data DNA relation information between the input data and the output data; and establishing, with respect to a plurality of stored data, a data DNA relation network among the plurality of stored data according to the data DNA relation information of each of the stored data.

In this method, for any one of processed data, the input data and the output data associated with the task logic may be determined by acquiring executing logic corresponding to the task logic. Because the task logic exists between the input data and the output data, it may be determined that the input data and the output data have a data DNA relation, and then, for each of the stored data, the data DNA relation among data may be determined through the method, and the data DNA relation network of all stored data may be acquired at last. Further, if certain data in the network is problematic, other suspected problematic data may be found through the data DNA relation network, so as to allow the technician to remove or modify data having quality problem, and improve the quality of the stored data.

In another embodiment of the disclosure, as illustrated in Figure 2, the step S103 illustrated in the above Figure 1 may include the following steps.

In step S1031: a node diagram including the plurality of stored data is generated.

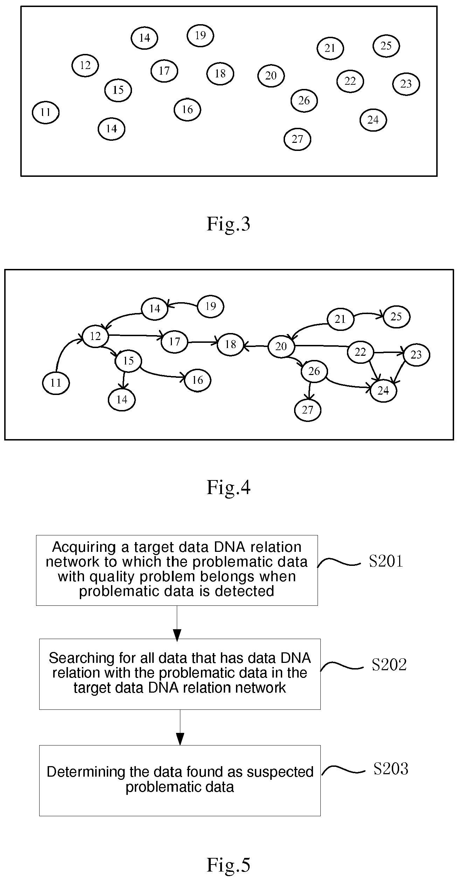

In the embodiment of the disclosure, each of the stored data is presented in form of node in the node diagram. As shown in Figure 3, each of the circles is a node and represents one datum. Each of the nodes is provided with a numeral corresponding to the data.

In stepS1032: whether the data DNA relation exists between any two of the nodes in the node diagram is determined.

In this step, the fact that whether a data DNA relation exists between the two nodes is determined by determining whether the two nodes have task logic.

If the data DNA relation exists between the two nodes, the step S1033 is performed. Otherwise, the process ends.

In step S1033: a data flow direction of the two nodes is indicated between the two nodes according to the data DNA relation information.

As illustrated in Figure 4, the data flow direction of the two nodes is indicated as arrow between the two nodes, thereby acquiring a data DNA relation network.

In other embodiment of the disclosure, as illustrated in Figure 5, after a data DNA relation network for a plurality of stored data is established, the method may further include the following steps.

In step S201: a target data DNA relation network to which problematic data with quality problem belongs is acquired, when the problematic data is detected.

After problematic data with quality problem is detected, a target data DNA relation network to which the problematic data belongs can be found by searching all data DNA relation networks established previously. Taking Figure 4 above as an example, when the problematic data is the data corresponding to a node 12, the data DNA relation network shown in Figure 4 may be determined as the target data DNA relation network.

In step S202: all data that has data DNA relation with the problematic data is searched for in the target data DNA relation network;

When the data corresponding to the node 12 in Figure 4 is the problematic data, all nodes that have task logic with the data is searched for. For example, nodes in Figure 4 which have direct task logic with the node 12 are: 11, 14 and 15; nodes in Figure 4 which have indirect task logic with the node 12 are: 14, 16, 17, 18 and 19.

In step S203: the data found is determined as suspected problematic data.

When the node 12 is used as output data, corresponding direct input data may include: a node 11 and a node 14. And with respect to the node 14, a node 19 is the direct input data of the node 14. When the node 12 is used as input data, corresponding direct output data may include: a node 15 and a node 17. And with respect to the node 15, corresponding direct output data may include the node 14 and a node 16; with respect to the node 17, corresponding direct output data may include a node 18.

Once the data corresponding to the node 12 is determined as the problematic data, because both the nodes 11, 14 and 15, and the nodes 14, 16, 17, 18 and 19 are associated with the node 12, these nodes may be regarded as suspected problematic data.

With the method according to the embodiments of the disclosure, in massive stored data, once a certain node is determined to have quality problem, all nodes associated with the node having quality problem can be found as suspected problematic data quickly through the data DNA relation network, so as to quickly locate the data with quality problem, and provide powerful guarantee for the solution of data problems.

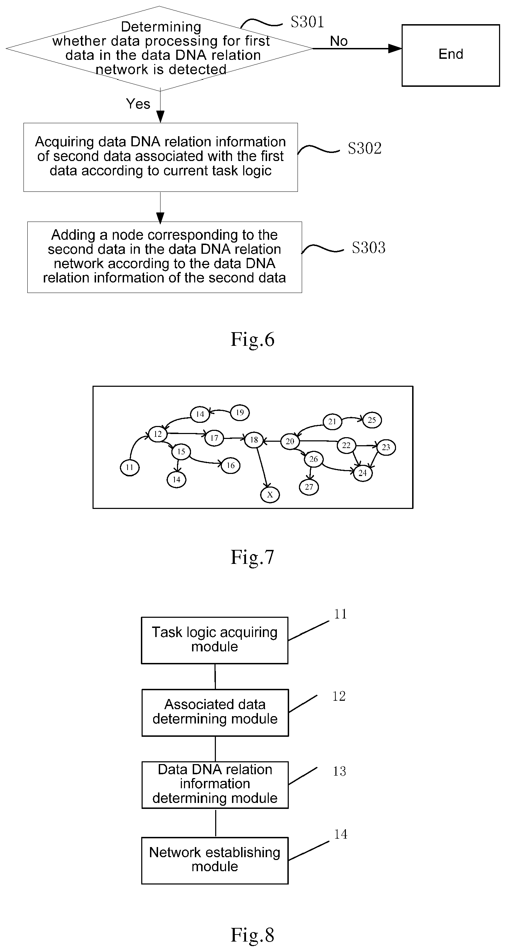

In another embodiment of the disclosure, the data corresponding to each of the nodes in the network may also be processed as input data. Accordingly, as shown in Figure 6, the method may further include the following steps.

In step S301: whether the data processing for first data in the data DNA relation network is detected, is determined.

With respect to data stored in the database, they may be called by the front end and used as the basis of data processing, that is, these stored data is logical operated as input data. Therefore, in this step, whether the data processing for first data in the data DNA relation network is performed, may be detected in real time.

If the data processing for a first data in the data DNA relation network is detected, step S302 is performed; otherwise, the process ends.

In step S302: the data DNA relation information of second data associated with the first data is acquired according to current the task logic.

When the first data as input data is processed, target data obtained after data processing may be regarded as the second data, and then the data DNA relation information between the first data and the second data is acquired.

In step S303: a node corresponding to the second data is added in the data DNA relation network according to the data DNA relation information of the second data.

Taking Figure 4 as example, when a node 18 is processed as input data, if generated second data is a node x, as shown in Figure 7, the node x may be added in Figure 4, and a data flow direction between the node 18 and node x is indicated.

Based on the same inventive conception, an embodiment of the disclosure also provides a device for data information processing. Figure 8 is a structural schematic illustrating a device for data information processing according to an embodiment of the disclosure. As shown in Figure 8, the device for data information processing may include: a task logic acquiring module 11, an associated data determining module 12, a data DNA relation information determining module 13 and a network establishing module 14.

The task logic acquiring module 11 acquires task logic of data processing, when the data is under processing.

In different storage media, data may be in different forms. For convenience of description, in the embodiments of the disclosure, the data is described in a form of data unit. Generally, the output target for each of data processing tasks may be regarded as one data unit. Further, the input source for each of data processing tasks may be regarded as one data unit. For example, in a relational database, each of the cells in a data table may be regarded as one data unit. Additionally, in a front-end display, the report form may be also regarded as one data unit.

Based on the above description, in this step, the task logic for data processing on a data unit may be acquired by taking the data unit as an object.

In embodiments of the disclosure, the task logic refers to data processing way, such as: data format conversion, data operation, etc.

The associated data determining module 12 determines input data and output data corresponding to the task logic according to executing logic of the task logic.

Each task logic has performing logic. In the embodiment of the disclosure, the executing logic refers to a flow direction between data. Taking data format conversion as an example, data before format conversion is the input data; data after format conversion is the output data. Taking data operation as an example, each of parameters before data operation may be the input data; and data after data operation is the output data.

The data DNA relation information determining module 13 determines the data DNA relation information of the input data and the output data.

Because both the input data and the output data are associated with the same task logic, the output data is acquired after data processing on the input data. Thus, it can be determined that correlation exists between the input data and the output data.

In the embodiment of the disclosure, the data DNA relation information includes at least a data flow direction.

The network establishing module 14 establishes, with respect to a plurality of stored data, a data DNA relation network among the plurality of stored data, according to the data DNA relation information of each of the stored data.

For each of the stored data recorded in a server, the data DNA relation information corresponding to the stored data can be acquired. As such, in the accumulate process of the stored data, the data DNA relation information of each of stored data can be acquired simultaneously. The flow direction relation between stored data may be acquired through the data DNA relation information. Therefore, in this step, on basis of the flow direction relation between stored data, relations among all stored data can be figured out to acquire the data DNA relation network among the plurality of stored data, that is, a "family tree" among data, the a parent node (that is the input data) and a child node (that is the output data) for each of the nodes may be found through the "family tree".

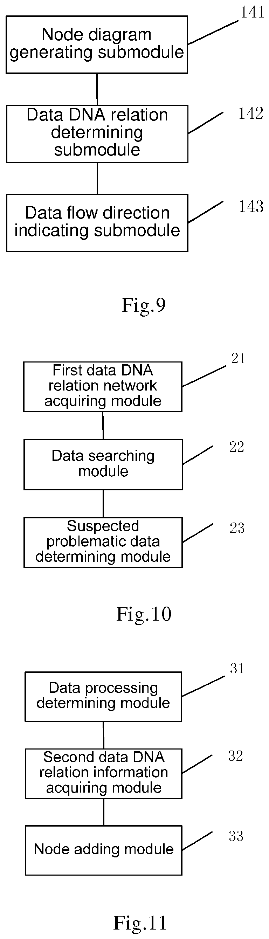

In another embodiment of the disclosure, as shown in Figure 9, the network establishing module 14 in the embodiment of Figure 8 above may include: a node diagram generating submodule 141, a data DNA relation determining submodule 142 and a data flow direction indicating submodule 143.

The node diagram generating submodule 141 generates a node diagram including the plurality of stored data, each of the stored data is presented in form of node in the node diagram.

In the embodiment of the disclosure, each of the stored data is presented in form of node in the node diagram. As shown in Figure 3, each of the circles is a node and represents one datum. Each of the nodes is provided with a numeral corresponding to the data.

The data DNA relation determining submodule 142 determines whether a data DNA relation exists between any two of the nodes in the node diagram.

In the embodiment of the disclosure, the fact that whether a data DNA relation exists between the two nodes may be determined by determining whether the two nodes have task logic.

The data flow direction indicating submodule 143 indicates a data flow direction of the two nodes between the two nodes according to the data DNA relation information if a data DNA relation exists between the two nodes.

As illustrated in Figure 4, the data flow direction of the two nodes is indicated as arrow between the two nodes.

In another embodiment of the disclosure, as shown in Figure 10, the device for data information processing according to the embodiment of the disclosure may further include: a first data DNA relation network acquiring module 21, a data searching module 22 and a suspected problematic data determining module 23.

The first data DNA relation network acquiring module 21 acquires a target data DNA relation network to which the problematic data with quality problem belongs when the problematic data is detected.

When problematic data with quality problem is detected, the target data DNA relation network to which the problematic data belongs can be found by searching all the data DNA relation networks established previously. Taking Figure 4 above as an example, when the problematic data is the data corresponding to a node 12, the data DNA relation network shown in Figure 4 may be determined as the target data DNA relation network.

The data searching module 22 searches for all data that has data DNA relation with the problematic data in the target data DNA relation network.

When the data corresponding to the node 12 in Figure 4 is the problematic data, all nodes that have task logic with the data may be searched for. For example, nodes in Figure 4 which have direct task logic with the node 12 are 11, 14 and 15; and nodes in Figure 4 which have indirect task logic with the node 12 are 14, 16, 17, 18 and 19.

The suspected problematic data determining module 23 determines the data found as suspected problematic data.

When the node 12 is used as output data, corresponding direct input data may include a node 11 and a node 14. And with respect to the node 14, a node 19 is the direct input data of the node 14. When node 12 is used as input data, corresponding direct output data may include a node 15 and a node 17. And with respect to the node 15, corresponding direct output data may include the node 14 and a node 16. With respect to the node 17, corresponding direct output data may include a node 18.

Once the data corresponding to the node 12 is determined as the problematic data, because both the nodes 11, 14 and 15, and the nodes 14, 16, 17, 18 and 19 are associated with the node 12, these nodes may be regarded as suspected problematic data.

With the device according to the embodiment of the disclosure, in massive stored data, once a certain node is determined to have quality problem, all nodes associated with the node having quality problem can be found as suspected problematic data quickly through the data DNA relation network, so as to quickly locate the data with quality problem, and provide powerful guarantee for the solution of data problems.

In still another embodiment of the disclosure, as shown in Figure 11, the device for data information processing according to the embodiment of the disclosure may further include: a data processing determining module 31, a second data DNA relation information acquiring module 32 and a node adding module 33.

The data processing determining module 31 determines whether data processing for first data in the data DNA relation network is detected.

With respect to data stored in the database, they may be called by the front end and used as the basis of data processing, that is, these stored data is logical operated as the input data. Therefore, in this step, whether the data processing for the first data in the data DNA relation network is performed, may be detected in real time.

The second data DNA relation information acquiring module 32 acquires the data DNA relation information of second data associated with the first data according to current task logic if the data processing for the first data in the data DNA relation network is detected.

When the first data as input data is processed, target data obtained after data processing may be regarded as the second data, and then the data DNA relation information between the first data and the second data is acquired.

The node adding module 33 adds a node corresponding to the second data in the data DNA relation network according to the data DNA relation information of the second data.

Taking Figure 4 as an example, when a node 18 is processed as input data, if the generated second data is a node x, as shown in Figure 7, the node x may be added in Figure 4, and the data flow direction between the node 18 and the node x is indicated.

According to the embodiment of the disclosure, a computer storage medium is also provided, wherein, the computer storage medium may be stored with program, some or all steps in the embodiments of the method for data information processing provided in the embodiments as illustrated in Figures 1-7 may be implemented when the program is executed.

The above description only illustrates detailed embodiments of the disclosure, and the protection scope of the disclosure is not limited to this. Those skilled in the art may easily think of various equivalent modifications or substitutions within technical scope disclosed in the disclosure, these modifications or substitutions shall be encompassed in the protection scope of the disclosure. Therefore, the protection scope of the disclosure shall be defined by the attached claims.

## Claims

1. A method for data information processing, comprising:
acquiring task logic of data processing when the data is under processing;
determining input data and output data corresponding to the task logic according to executing logic of the task logic;
determining data DNA relation information between the input data and the output data; and
establishing, with respect to a plurality of stored data, a data DNA relation network among the plurality of stored data, according to the data DNA relation information of each of the stored data.

2. The method according to claim 1, wherein, the data DNA relation information comprises at least: a data flow direction; and
the task logic comprises any one of data format conversion and data operation.

3. The method according to claim 1, wherein the establishing the data DNA relation network among the plurality of stored data comprises:
generating a node diagram comprising the plurality of stored data, each of the stored data being presented in form of node in the node diagram;
determining whether a data DNA relation exists between any two of the nodes in the node diagram; and
indicating a data flow direction of the two nodes between the two nodes according to the data DNA relation information if the data DNA relation exists between the two nodes.

4. The method according to claim 1, wherein the method further comprises:
acquiring a target data DNA relation network to which problematic data with quality problem belongs when the problematic data is detected;
searching for all data that has data DNA relation with the problematic data in the target data DNA relation network; and
determining the data found as suspected problematic data.

5. The method according to claim 1, wherein the method further comprises:
determining whether data processing for first data in the data DNA relation network is detected;
acquiring data DNA relation information of second data associated with the first data according to current task logic if the data processing for the first data in the data DNA relation network is detected; and
adding a node corresponding to the second data in the data DNA relation network according to the data DNA relation information of the second data.

6. A device for data information processing, comprising:
a task logic acquiring module, configured to acquire task logic of data processing when the data is under processing;
an associated data determining module, configured to determine input data and output data corresponding to the task logic according to executing logic of the task logic;
a data DNA relation information determining module, configured to determine data DNA relation information between the input data and the output data; and
a network establishing module, configured to establish, with respect to a plurality of stored data, a data DNA relation network among the plurality of stored data according to the data DNA relation information of each of the stored data.

7. The device according to claim 6, wherein, the data DNA relation information comprises at least: a data flow direction; and
the task logic comprises any one of data format conversion and data operation.

8. The device according to claim 6, wherein, the network establishing module comprises:
a node diagram generating submodule, configured to generate a node diagram comprising the plurality of stored data, each of the stored data is presented in form of node in the node diagram;
a data DNA relation determining submodule, configured to determine whether a data DNA relation exists between any two of the nodes in the node diagram; and
a data flow direction indicating submodule, configured to indicate a data flow direction of the two nodes between the two nodes according to the data DNA relation information, if the data DNA relation exists between the two nodes.

9. The device according to claim 6, wherein, the device further comprises:
a first data DNA relation network acquiring module, configured to acquire a target data DNA relation network to which problematic data with quality problem belongs when the problematic data is detected;
a data searching module, configured to search for all data that has data DNA relation with the problematic data in the target data DNA relation network; and
a suspected problematic data determining module, configured to determine the data found as suspected problematic data.

10. The device according to claim 6, wherein the device further comprises:
a data processing determining module, configured to determine whether data processing for first data in the data DNA relation network is detected;
a second data DNA relation information acquiring module, configured to acquire data DNA relation information of second data associated with the first data according to current task logic if the data processing for the first data in the data DNA relation network is detected; and
a node adding module, configured to add a node corresponding to the second data in the data DNA relation network according to the data DNA relation information of the second data.
